# EUROPEAN PATENT APPLICATION

(11) **EP 1 090 607 A1**
(43) Date of publication of application: **11.04.2001**
(21) Application number: 00119759.9
(22) Date of filing: 11.09.2000
(51) Int. Cl.: A61C 19/00

(54) **A dental handpiece for the polymerization of photosetting compounds or resins**

(30) Priority: 08.10.1999 EP 99830631
(71) Applicant: Mectron S.R.L., 16042 Carasco (Genova) (IT)
(72) Inventor: Bianchetti, Fernando, 16043 Chiavari (Genova) (IT); Vercellotti, Domenico, Lupara - Sestri Levante (Genova) (IT)
(74) Representative: Petruzziello, Aldo

(57) **Abstract**

A dental handpiece (1, 100) for the polymerization of photosetting compounds or resins which includes a casing (2) inside which is positioned an electronic circuit (13) which powers a light source (7) including a plurality of semiconductor dies (31) mounted in truncated conical reflecting housings (30) in a metal support (32) to obtain LEDs that emit blue light allowing the polymerization of said photosetting resins; such electronic circuit (13) includes a switch (18) with a button (19) which, when pressed by an operator, controls the turning on and/or turning off of LEDs (31), a system clock (21) to keep the LEDs lit for a preset time period and an acoustic signal generator (20) to signal the turning off of the LEDs.

## Description

The present invention concerns a dental handpiece for the polymerization of photosetting compounds or resins.

The invention refers, in particular, to a tool used by dentists for the polymerization of photosetting compounds or resins especially utilised in dental protheses.

Photosetting resins are applied, in a semifluid state, to patients' dental prostheses, and then hardened by a polymerization process. Such a polymerization process involves heating the resins for a certain length of time, using a light source that emits blue light, i.e. having an emission spectrum with a wavelength of about 470 nm.

At present, dental handpieces, according to the prior art, for such purposes, use halogen lamps as the light source. The light emitted by the halogen lamps is filtered by dichroic filters, so that a blue light with a light spectrum having a wavelength between 430 and 510 nm is obtained. The light emerging from the dichroic filters is transported, through optic fibres, to the outlet of the dental handpiece, enabling the dentist to direct it on the resin to be polymerised, applied to the patient's dental prosthesis.

The known dental handpieces utilizing halogen lamps as polymerizer lamps have various drawbacks.

In order to achieve polymerization of the resin, the light emerging from the dental handpiece must have a power output of approximately 500 mW. As halogen lamps have a very low efficiency, intended as power output to power input ratio, (efficiency value η = 0.5-1%), this results in high energy dissipation, by the polymerizer lamps, that reach values of 50 to 100W. Consequently, excessive overheating of the dental handpiece results and hence problems for heat dissipation or removal. In fact, to assist with heat removal, it is necessary to use large dental handpieces that may be equipped with internal ventilation systems.

As a consequence, such dental handpieces are excessively bulky and heavy, this being detrimental to their manoeuvrability.

Besides, with the power output provided by halogen lamps, fairly long resin polymerization times are envisaged, which is very annoying, both for the patient undergoing treatment and the dentist who must carry it out.

In an attempt to try to reduce resin polymerization times, dental handpieces, with different light sources, have appeared on the market. Dental handpieces are per se known that use gas discharge lamps (plasma torches) as a light source which emit high power white light, that is filtered so as to obtain a light beam with an emission spectrum centred on blue, as dental handpieces are also known, using a laser light source, that directly transmit a light beam with an emission spectrum centred on blue.

With this type of dental handpiece it is possible to obtain a light beam with a power output approximately ten times greater compared to that obtained with halogen lamps, therefore shorter polymerization times are requested. However, such dental handpieces, apart from being very expensive, have the problem of needing to dissipate a large amount of heat, and inevitably must provide a heat dissipation or cooling system making them excessively bulky and heavy.

All the known dental handpieces using halogen lamps or plasma torches or laser as a light source have an electricity supply system reserved only for the electricity supply of such a light source. This makes the dental handpiece bulky and not versatile, because of the purpose-built circuitry provided for such light sources.

An optic irradiation device is described in patent application PCT/GB 98/02905 suitable for polymerization, using as a light source, a cluster of light emitting diodes (LEDs) of the type commonly available on the market, i.e. including a semiconductor junction covered by a covering capsule made of transparent material.

In order to increase the number of LEDs in the cluster, the covering capsule of each LED is faceted, so as to bunch the LEDs sideways to take up minimum space. Such a device has the drawback that a large number of LEDs is necessary to focus a beam of light which is suitable for polymerization. Therefore, to put as many LEDs into a confined space, as in a dental handpiece, it is necessary to make facets on the protection capsule of each LED, with a subsequent wasting of time and costs due to the extra work. In spite of this, the proposed LED clusters are excessively bulky.

Patent application PCT WO 99/35995 describes a device for polymerization by means of radiation using as its light source an array of LED dies mounted on a printed circuit board.

Dies are parallelepipid-shaped plates or chips without any protection capsule. The dies consist of a semiconductor junction that emits light when biassed. In this solution, although a die emits light from six faces, only the light emission from the upper face is exploited.

In fact, the dies are mounted on a printed circuit board with a planar shape whereon metal pads and tracks provided for the electrical connection prevent propagation of the light emission coming from the lateral faces of the dies. Consequently the power available is about one fifth of that provided by said dies. As a result, in order to reach a power sufficient to obtain the polimerization, the device according to patent application WO 99735995 requires a very large number of dies (about 100).

This leads to various disadvantages. In fact there are complications in housing such a large number of dies on a printed circuit board of limited size. Furthermore, since the luminous efficiency of each die is not best exploited, there is high heat formation due in part to the luminous energy of the dies that is not exploited. This leads to considerable practical disadvantages in designing adequate heat dissipators that allow operation of the device.

The aim of the invention is to eliminate such drawbacks by providing a dental handpiece, for the polymerization of photosetting compounds or resins, of reduced dimensions and bulk, proving versatile, economic and easy to produce.

This aim is reached, in accordance with the invention, with the characteristics listed in the enclosed claim 1.

The preferred embodiments of the invention emerge from the dependent claims.

In the dental handpiece for the polymerization of photosetting compounds or resins, the light source consists of a plurality of semiconductor chips or square dies, with a side dimension of less than 1 mm.

The material utilised is preferably gallium arsenide (GaAs), although silicon or another semiconductor may also be used.

Each die has a p-n junction, i.e. a junction between two semiconductor layers doped p-type and n-type, respectively. The p-type layer is obtained through the implantation of electron acceptor elements, with the n-type layer being obtained through the implantation of electron donor elements.

When a p-n junction is directly biased, photons are emitted with consequent light radiation. Therefore each single die acts like an LED (light emitting diode).

The p-n junction of each die is made in such a way as to generate an emission of photons that transmit a blue light with an emission spectrum perfectly centred on 470 nm.

Each die is directly assembled bare inside respective truncated conical recesses in a polished metal support, without using any container or protection capsule. In this manner, said recesses reflect the light emission of all the faces of the dies in the direction of use, with an increase in luminous efficiency of more than 400%. A printed circuit board with holes registrated with the reflecting recesses is fixed on this metal support. In this manner the terminals of the dies can be soldered to pads provided on the printed circuit for the electrical connection of the dies. The printed circuit board and metal support are sandwiched between a heat dissipator and an optical system for adequate focusing of the individual light beams.

The advantages of this solution are apparent in that it succeeds in exploiting the luminous efficiency of the LED dies to the utmost. In fact with a system according to the invention a smaller number of LED dies can be used, with a consequent saving in space occupied and heat dissipation systems.

The LEDs have a very high efficiency (η= 40%), around forty times greater compared to halogen lamps; as a result the LED power dissipation is very low and therefore problems of heat dissipation and excessive overheating of the dental handpieces are absent. Hence, complicated cooling systems become unnecessary and therefore dental handpieces of much reduced size and weight proving more manageable may be produced.

The utilisation of LEDs as a light source, allows the electrical supply of a dental chair unit to be used as the electricity supply for such a light source. The dental chair unit is a unit used by dentists which includes an adjustable reclining seat on which the patient sits, together with a series of dental instruments, e.g. turbines, micromotors, piezoelectric scalers, etc. This, apart from reducing the dental handpiece's bulk, since it is not necessary to develop circuitry expressly for supplying its light source, makes the same dental handpiece very versatile and adaptable to the various instruments normally used in dentistry. Since LEDs dissipate very limited power, the dental handpiece, according to the invention, may also be fed autonomously by batteries, thus avoiding the need for connectors and mains wires and therefore making the dental handpiece autonomous and easily transportable.

Further characteristics of the invention will be made clearer from the detailed description that follows, referring to its purely exemplary forms and therefore non limiting embodiments thereof, shown in the appended drawings, in which:
Fig. 1 shows a part sectional view of a first embodiment of the dental handpiece, according to the invention;
Fig. 2 shows a part sectional view of a second embodiment of the dental handpiece according to the invention;
Figure 3 is a perspective exploded view of the illumination device of the handpiece according to the invention;
Figure 4 is a perspective exploded view like Figure 3, taken from another angle;
Figure 5 is a plan view from above of the illumination device in Figure 4;
Figure 6 is an axial section of the illumination device taken along the plane of section VI-VI in Figure 5;.
Figure 7 is an enlarged view of the detail in the circle A in Figure 6;
Figure 8 is a perspective view, illustrating the detail in Figure 7.

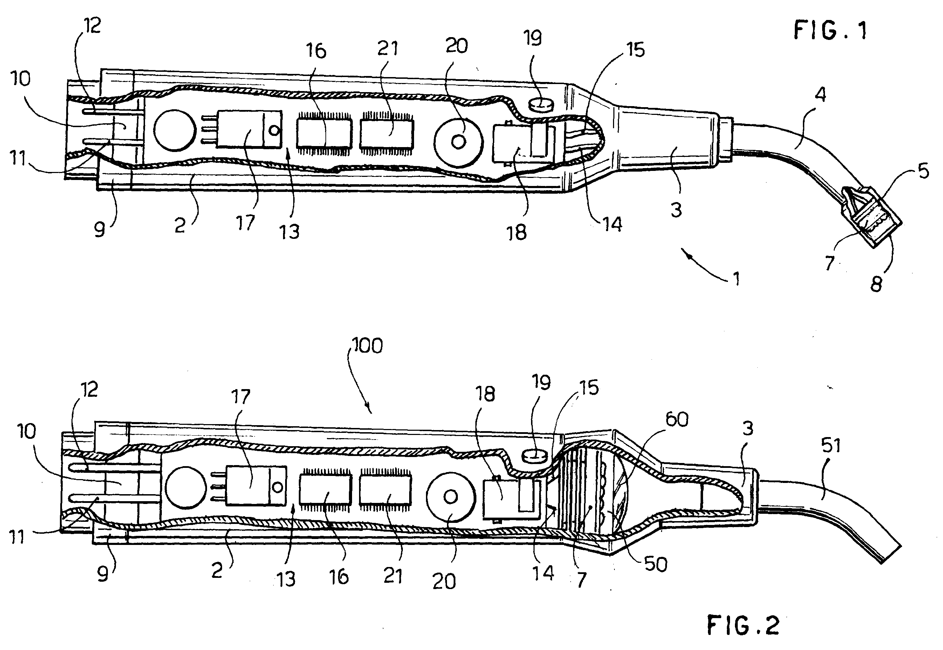

In Fig. 1, a first embodiment of a dental handpiece, according to the invention is shown as a whole, and referenced 1. Dental handpiece 1 includes a casing 2 of basically hollow cylindrical shape with a cylindrical tang 3 ending in a slightly bent cylindrical tube or pipe 4. At the end of tube 4, a housing 5 containing a light source 7 is located.

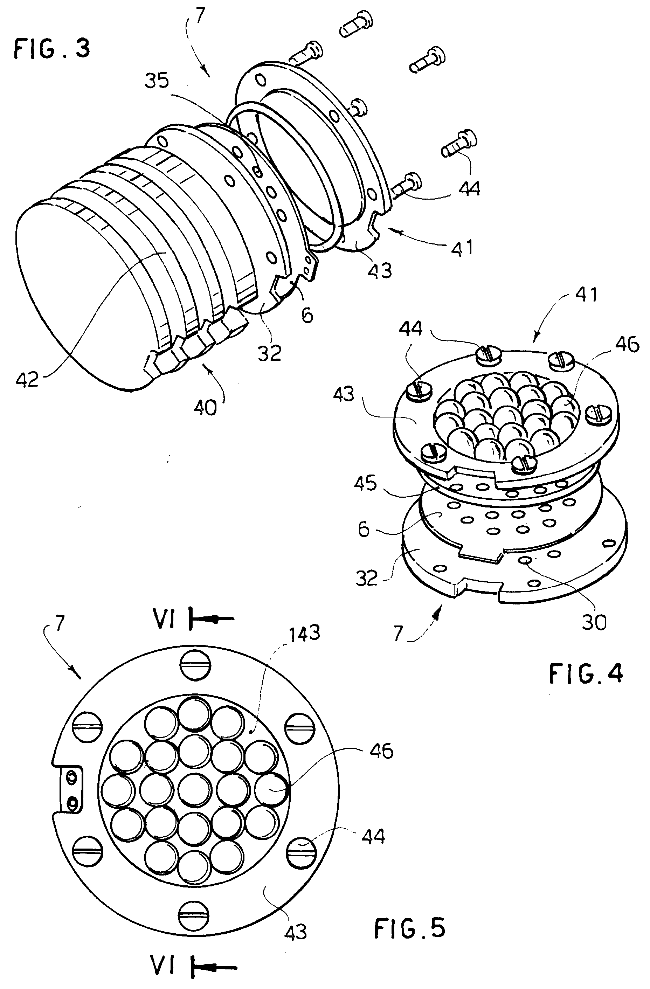

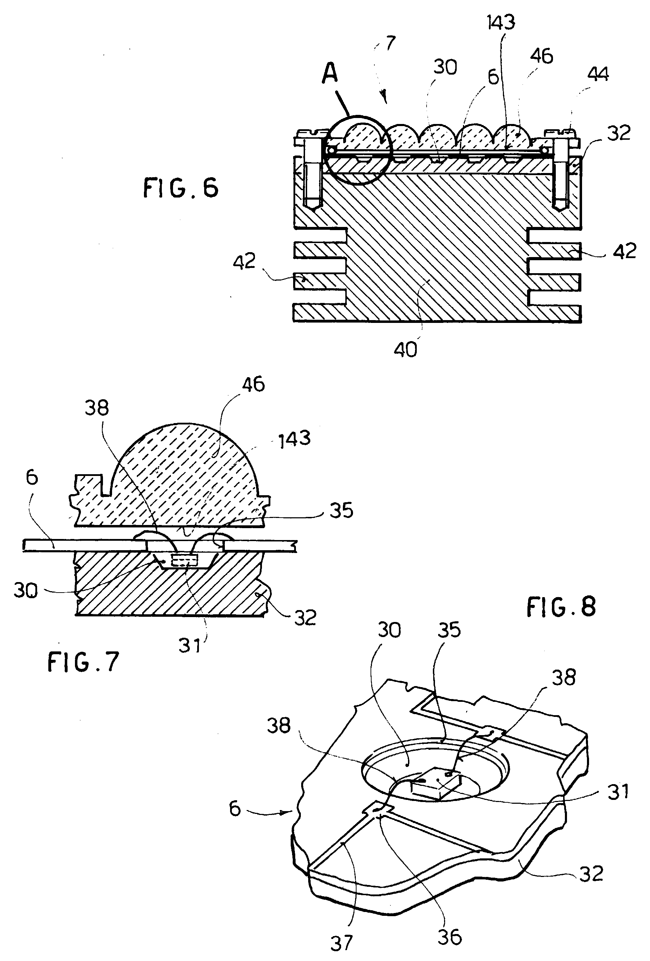

As shown in Figs. 3-8, the light source 7 comprises a plurality of semiconductor chips or dies 31 The semiconductor material utilised is preferably gallium arsenide.

The single die 31 is basically square shaped with a side dimension of less than 1 mm.

Die 31 is produced by a two layered semiconductor p-n junction, a p-type doped layer with electron acceptor materials and a n-type layer doped with electron donor materials.

When directly biased a p-n junction generates an emission of photons with consequent light radiation, therefore each die 31 behaves like an LED (light emitting diode).

Each die 31 is mounted in a housing 30 of a support 32. The housings 30 have a substantially truncated conical shape with reflecting inside walls. The shape of the walls of the housings 30 is such as to reflect the light emitted by all faces of the die 31 towards the direction of use. In this manner the luminous efficiency is increased by about 400% compared with that of a die mounted on a flat support.

The support 32 can be made of reflective metal. Alternatively the support 32 can be made of another material and the internal walls of the housings 30 are coated with a reflective material.

A printed circuit board 6 having a plurality of holes 35 level with the housings 30 is positioned above the printed circuit board 6. Pads 36 for the electrical contacts and electrical connection tracks 37 are provided above the printed circuit board 6.

The terminals 38 of each die are connected by means of soldering to the respective pads 36. In this manner suitable electrical connections of the dies can be made. In the specific case a parallel connection of three series of dies is used, wherein each series comprises seven dies connected in series. In this manner there is a light source comprising a total of twenty-one dice 31 which is sufficient for the purposes of the handpiece according to the invention.

The support 32 and the printed circuit 6 are mounted sandwiched between a heat dissipator 40 and an optical system 41. The heat dissipator 40 is cylindrical in shape and has protruding fins 42 shaped like annular plates.

The optical system 41 provides an annular support plate 43 wherein holes are provided to receive bolts 44 for fixing to the support 32 and to the dissipator 40. Between the plate 43 of the optical system 41 and the support 32 is interposed a gasket 45 of the O-ring type which surrounds the edge of the printed circuit 6.

The plate 43 of the optical system 41 supports the optical system proper consisting of a plurality of substantially dome-shaped focussing lenses 46, made of transparent material and disposed on a base 143 of the same transparent material. When the optical system is mounted, the focussing lenses 46 are registered with the respective housings 30 of the dies 31, that is to say the axis of each die coincides with the axis of each focussing lens 46.

Alternatively, instead of providing a base 143, the focussing lenses 46 can be mounted separately directly above the housings 30 of the dies.

The lenses 46 of the optical system 41 allow adequate focussing of the individual light beams coming from the dies 31 and reflected on the walls of the housings 30.

In the present embodiment of the invention, the dies or LEDs 31 are located in housing 30 in a linear array configuration, symmetrically arranged in parallel lines and slightly separated from each other. In particular, three arrays of five dies each are provided and two end arrays of three dies each, giving a total of twenty-one dice.

The LEDs 31 used emit blue light having a spectrum with a wavelength of 470 nm.

An end of housing 5 is covered by a small glass 8 to protect the LEDs 31 and to let the light emitted by such LEDs pass through.

At the bottom end of the casing 2 of the dental handpiece is a cover 9 inside which is an electrical connector element 10 with electrical contacts 11 and 12 for the electricity supply and earthing of the dental handpiece internal circuitry. The connector 10 may be connected to a complementary connector element to be powered from mains supply or a dental chair unit. The dental chair unit is equipped with electric transformers which draw power supply from the mains supply and provide a voltage supply of 24V a.c. (alternating current) or 32V d.c. (direct current). Dental handpieces are also available without a connector and with an autonomous power supply such as batteries, capable of providing direct current voltage supply suitable for powering the printed circuit board 6 containing the LEDs 31.

The electrical contacts 11 and 12 of connector 10 are connected to an electronic circuit 13 situated in the casing 2 of the dental handpiece. Two electrical wires 14 and 15 are connected to the electronic circuit 13 for the power supply and earthing of the printed circuit board 6. The electrical wires 14 and 15 pass through the tang 3 and tube 4 and are connected to the contacts 40 of the printed circuit board 6 containing the LEDs 31.

If the LEDs 31 are connected in series, in order to function properly, the electronic circuit 13 must be able to supply the LEDs with a constant current of 20 mA and a voltage equal to the number of LEDs multiplied by the bias voltage of each LED, i.e. about 3.3-3.5 V. Clearly, LED connections in parallel may be made or in parallel-series, to provide a lower voltage drop and a greater supply current.

The electronic circuit 13 includes a rectifier 17 and a current generator 16. If an alternating current voltage signal is drawn from the dental chair unit, this signal is then passed through the rectifier 17 in which it is levelled off and sent to the current generator 16. The current generator 16 should preferably be a PWM type generator (pulse width modulation), which is able to increase or decrease the supply voltage to be sent to the printed circuit board 6, depending on the number of LEDs 31 and their connection.

Besides, the electronic circuit 13 includes a switch 18, a system clock 21 and a generator of acoustic signal or buzzer 20. Switch 18 is controlled by a button 19 projecting out of the casing 2 of the dental handpiece, so that it can be pressed by an operator. When the operator presses button 19, switch 18 turns on supply to LEDs 7, which then start emitting blue light. At the same time, the system clock 21 begins counting for a preset time period (e.g. 20 seconds). Upon termination of this time period, the electricity supply of the printed circuit board 6 is turned off and therefore LEDs 31 turn off and, simultaneously, buzzer 20 emits an acoustic sound signalling the turning off of LEDs 31. Pressing button 19 during the operation cycle causes the turning off of LEDs 31 and resets the system clock 21, for the next cycle. The acoustic signalling device can obviously be replaced by a visual signalling device, without however departing from the scope of the invention.

A second embodiment of a dental handpiece 100 according to the invention is shown in figure 2, in which identical or similar parts are denoted by the same reference numbers used for the first embodiment.

In the dental handpiece 100, the light source 7 is positioned in a housing 50, formed between the front end of casing 2 of the dental handpiece and tang 3. In this embodiment, around twenty LEDs 31 should preferably be used.

In housing 50, at a suitable distance from the LED matrix 31, a condenser lens 60 is positioned whose purpose is to focus the light emitted by LEDs 31 towards an optic fibre 51. The optic fibre 51 is engaged to the end of tang 3 and is of a slightly bent cylindrical shape so as to effectively direct the blue light towards the patient's oral cavity. Moreover, the optic fibre has the advantage of being able to be sterilized in an autoclave at high temperatures.

## Claims

1. A dental handpiece (1, 100) for the polymerization of photosetting compounds or resins, including a casing (2) inside which an electronic circuit (13) is located to provide power supply for a light source (7) formed by LEDs (31) in order to allow the polymerization of said photosetting resins, characterised in that said LEDs (31) are produced with a plurality of dies (31) of semiconductor mounted into respective housings (30) with reflecting walls provided on a support.

2. A dental handpiece according to claim 1, characterised in that the inside walls of said housings (30) define a recess with a substantially truncated conical shape.

3. A dental handpiece according to claim 1 or 2, characterised in that said support (32) is made of reflecting metal material.

4. A dental handpiece according to claim 1, characterised in that said semiconductor dies (31) are produced with a p-n junction of gallium arsenide.

5. A dental handpiece according to any one of the previous claims, characterised in that said dies (31) are basically square shaped with a side dimension of less than one millimetre.

6. A dental handpiece according to any one of the previous claims, characterised in that above said support (32) housing the dies (31) is disposed a printed circuit (6) having a plurality of holes (35) in register with said housings (30), pads (7) and tracks (37) being provided on said printed circuit for electrical connection of said dies (31).

7. A dental handpiece according to any one of the previous claims, characterised in that said light source (7) comprises an optical system (41) disposed above said dies (31) comprising a plurality of focussing lenses (46) disposed in register with said dies (31).

8. A dental handpiece according to any one of the previous claims, characterised in that said light source (7) provides a heat dissipator (40) disposed below said housing support (32) of the dies (31).

9. A dental handpiece according to any one of the previous claims, characterised in that said LEDs (31) emit blue light with an emission spectrum centred around 470 nm.

10. A dental handpiece (1) according to any one of the previous claims, characterised in that said light source (7) is located in a housing (5) inside the dental handpiece (1) in its top end, said housing (5) being covered and protected by a small glass (8) which lets light pass through.

11. A dental handpiece (100) according to any one of the claims from 1 to 6, characterised in that said light source (7) is positioned in a housing (50) within the casing (2) of the dental handpiece and the light coming from said LEDs (31) is focused by a condenser lens (60).

12. A dental handpiece (100) according to claim 11, characterised in that the light emitted by LEDs (31) and focused by the condenser lens (60) is sent towards an optic fibre (51) whose purpose is to direct it towards the patient's oral cavity.

13. A dental handpiece according to any one of the previous claims, characterised in that a connector element (10) is connected to said electronic circuit (13) able to join up with a complementary connector element to draw power supply from the mains supply or from a dental chair unit.

14. A dental handpiece according to any one of the previous claims, characterised in that an independent system for the dental handpiece power supply, including batteries, is provided.

15. A dental handpiece, according to any one of the previous claims, characterised in that said electronic circuit (13) includes a rectifier (17) able to rectify the alternating current voltage drawn from the mains supply or from a dental chair unit and a current generator (16) that draws voltage from said rectifier (17) and supplies an appropriate current to the printed circuit board (6) for the bias of LEDs (31).

16. A dental handpiece according to claim 12, characterised in that said current generator is a PWM type generator (Pulse Width Modulation) able to increase or decrease the voltage to be supplied to the printed circuit board (6) depending on the number of LEDs (31) and their connection.

17. A dental handpiece according to any one of the previous claims, characterised in that said electronic circuit (13), includes a switch (18) connected to a button (19) which, when pressed by an operator, controls the turning on and/or turning off of said LEDs (31) assembled on the printed circuit board (6).

18. A dental handpiece according to any one of the previous claims, characterised in that said electronic circuit (13) includes a system clock (21) that keeps said LEDs (31) lit for a preset time period.

19. A dental handpiece according to any one of the previous claims, characterised in that said electronic circuit (13) includes an acoustic and/or visual signal generator (20) capable of emitting an acoustic and/or visual signal, when said LEDs (31) are turned off.
